(19) Europäisches Patentamt
European Patent Office
Office européen des brevets

(11) EP 4 732 816 A1

(12) EUROPEAN PATENT APPLICATION
published in accordance with Art. 153(4) EPC

(43) Date of publication:
29.04.2026 Bulletin 2026/18

(51) International Patent Classification (IPC):
A61F 13/15 (2006.01)    A61F 13/53 (2006.01)

(21) Application number: 24825993.9

(52) Cooperative Patent Classification (CPC):
A61F 13/15; A61F 13/53

(22) Date of filing: 20.06.2024

(86) International application number:
PCT/JP2024/022474

(87) International publication number:
WO 2024/262592 (26.12.2024 Gazette 2024/52)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 20.06.2023 JP 2023100620

(71) Applicant: Sumitomo Seika Chemicals Co., Ltd.
Kako-gun, Hyogo 675-0145 (JP)

(72) Inventor: YAMAMOTO, Tomoe
Himeji-shi, Hyogo 672-8076 (JP)

(74) Representative: Mewburn Ellis LLP
Aurora Building
Counterslip
Bristol BS1 6BX (GB)

(54) DEODORANT COMPOSITION

(57) There is provided a deodorant composition having an excellent deodorizing effect against methyl mercaptan. A deodorant composition comprising a porous deodorant comprising a halogen; and water-absorbent resin particles, wherein a content of the halogen is 0.0030% by mass or more.

FIG. 1

**Description**

Technical Field

The present invention relates to a deodorant composition.

Background Art

An absorbent article, such as a disposable diaper, a sanitary napkin, or an incontinence pad, is composed of an absorbent material that absorbs and retains a body liquid, such as urine or menses excreted from the body, the absorbent material being positioned mainly in a central portion, a liquid-permeable front sheet (top sheet) positioned on the side of the absorbent article that is brought into contact with the body, and a liquid-impermeable rear sheet (back sheet) positioned opposite to the side that is brought into contact with the body. The absorbent material is typically composed of hydrophilic fibers, such as pulp, and a water-absorbent resin.

Citation List

Patent Literature

Patent Literature 1: JP-A-2001-323155

Summary of Invention

Technical Problem

A porous deodorant, such as an activated carbon, may be used in the absorbent article for the purpose of deodorizing odors emitted from body fluids such as urine.
The absorbent article retains not only body fluids such as urine but also feces, for example. The odor of methyl mercaptan is emitted from feces.
Porous deodorants containing halogens are known as deodorants that exhibit an excellent deodorizing effect against sulfur compounds such as methyl mercaptan. Such a porous deodorant comprising a halogen exhibits the effect of reducing the odor from a sulfur compound, through reaction of the halogen with the sulfur compound.
The inventor of the present invention conducted research on using a porous deodorant comprising a halogen to impart a deodorizing effect against methyl mercaptan to an absorbent article. As a result, the inventor has found a new problem, namely that although the odor of methyl mercaptan is reduced by the porous deodorant comprising a halogen, the deodorizing effect against the odor of methyl mercaptan is attenuated in the presence of urine together with mercaptan.
It is a main object of the present invention to provide a deodorant composition having an excellent deodorizing effect against methyl mercaptan.

Solution to Problem

The present inventor has conducted extensive research to solve the aforementioned problem. As a result, the present inventor has found that when a porous deodorant comprising a halogen and water-absorbent resin particles are used in combination in a deodorant composition, and the content of the halogen in the deodorant composition is set to equal to or more than a predetermined value, such a deodorant composition exhibits an excellent deodorizing effect against methyl mercaptan. The present invention has been accomplished as a result of further research based on this finding.
In summary, the present invention provides aspects of the invention comprising the following features:

Item 1. A deodorant composition comprising:

a porous deodorant comprising a halogen; and
water-absorbent resin particles,
wherein a content of the halogen is 0.0030% by mass or more.

Item 2. The deodorant composition according to item 1, wherein the halogen comprises at least one selected from the group consisting of bromine and chlorine.
Item 3. The deodorant composition according to item 1 or 2, wherein the porous deodorant comprises at least one compound selected from the group consisting of activated carbons, zeolite, silicon dioxide, and silicates.

Item 4. The deodorant composition according to any one of items 1 to 3, wherein a ratio (Z/(X + Y)) of a content Z (% by mass) of the water-absorbent resin particles to a sum (X + Y) of a content X (% by mass) of the porous deodorant and the content Y (% by mass) of the halogen is 200 or more.

Item 5. The deodorant composition according to any one of items 1 to 4, wherein the water-absorbent resin particles have a water absorption rate of 50 seconds or less, as measured by a Vortex method.

Item 6. An absorbent article comprising the deodorant composition according to any one of items 1 to 5.

Advantageous Effects of Invention

According to the present invention, it is possible to provide a deodorant composition having an excellent deodorizing effect against methyl mercaptan.

Brief Description of Drawings

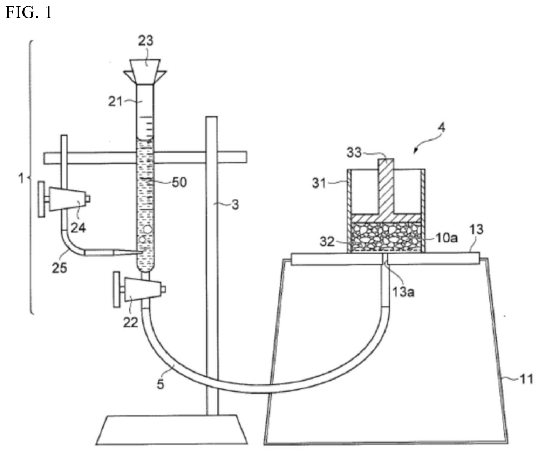

Fig. 1 is a schematic diagram of a measurement apparatus for physiological saline absorption capacity under a load of 4.14 kPa.

Description of Embodiments

As used herein, the term "comprising" includes "consisting essentially of" and "consisting of". As used herein, the term "(meth)acrylic" refers to "acrylic or methacrylic", and the term "(meth)acrylate" refers to "acrylate or methacrylate". As used herein, the term "water-soluble" refers to having a water solubility of 5% by mass or more at 25°C.

As used herein, values connected with "to" refer to the numerical range including the values before and after "to" as the lower and upper limits. When a plurality of lower limits and a plurality of upper limits are mentioned separately, any lower limit and any upper limit may be selected and connected with "to".

1. Deodorant Composition

A deodorant composition of the present invention comprises a porous deodorant comprising a halogen; and water-absorbent resin particles, wherein a content of the halogen is 0.0030% by mass or more. The deodorant composition of the present invention comprising these features exhibits an excellent deodorizing effect against methyl mercaptan. The deodorant composition of the present invention will be hereinafter described in detail.

As described above, the present inventor has conducted research on using a porous deodorant comprising a halogen to impart a deodorizing effect against methyl mercaptan to an absorbent article. However, the inventor has found a new problem, namely that although the odor of methyl mercaptan is reduced by the porous deodorant comprising a halogen, the deodorizing effect against the odor of methyl mercaptan from feces is attenuated in the presence of urine together with methyl mercaptan.

The present inventor has conducted further research to find that a main cause of this problem is that polyvalent metal salts (alkali metals and alkaline earth metals) contained in urine react with the halogen of the porous deodorant, which attenuates the deodorizing effect against methyl mercaptan, and prevents sufficient deodorization of the methyl mercaptan contained in feces. Furthermore, the present inventor has found that an excellent deodorizing effect against methyl mercaptan can be exhibited by using a porous deodorant comprising a halogen and water-absorbent resin particles in combination in a deodorant composition, and setting the content of the halogen in the deodorant composition to 0.0030% by mass or more. It can be assumed that in the deodorant composition of the present invention, polyvalent metal salts contained in urine are absorbed by the water-absorbent resin particles before contacting the halogen molecules of the porous deodorant, which prevents the reaction between the halogen of the porous deodorant and the polyvalent metal salts, so that the deodorant composition of the present invention exhibits a high deodorizing effect against methyl mercaptan contained in large amounts in feces.

(Porous Deodorant Containing Halogen)

The porous deodorant comprising a halogen is a porous deodorant that contains a halogen element.

In the present invention, the porous deodorant comprising a halogen may be any known porous deodorant that exhibits a deodorizing effect against methyl mercaptan.

In view of satisfactorily achieving the effect of the present invention, in the porous deodorant comprising a halogen, the halogen is preferably present as a halogen molecule in the porous deodorant. The halogen preferably comprises at least one selected from the group consisting of bromine and chlorine, and more preferably comprises bromine.

A preferred specific example of the porous deodorant comprising a halogen is an activated carbon comprising bromine. For example, a bromine-impregnated activated carbon is known as a porous deodorant having a deodorizing effect against sulfur compounds, and can be used as the porous deodorant comprising a halogen in the present invention. Examples of the bromine-impregnated activated carbon include the activated carbon disclosed in JP-A-H11-33397. Methods of impregnating an activated carbon with bromine are known for the bromine-impregnated activated carbon, including, for example, a vapor-phase impregnation method in which a carrier gas containing bromine gas is contacted with an activated carbon that has undergone a desired treatment; a liquid-phase impregnation method in which a treated activated carbon is immersed in aqueous bromine; and a method in which liquid bromine or the like is directly sprayed on a treated activated carbon.

In the deodorant composition of the present invention, the content of the halogen is 0.0030% by mass or more. In view of more satisfactorily achieving the effect of the present invention, the content of the halogen in the deodorant composition is preferably 0.0035% by mass or more, more preferably 0.0040% by mass or more, and still more preferably 0.0050% by mass or more, while the content of the halogen is preferably 0.010% by mass or less, more preferably 0.0080% by mass or less, and still more preferably 0.0070% by mass or less. Preferred ranges include from 0.0030 to 0.010% by mass, from 0.0030 to 0.008% by mass, from 0.0030 to 0.007% by mass, from 0.0035 to 0.010% by mass, from 0.0035 to 0.008% by mass, from 0.0035 to 0.007% by mass, from 0.0040 to 0.010% by mass, from 0.0040 to 0.008% by mass, from 0.0040 to 0.007% by mass, from 0.0050 to 0.010% by mass, from 0.0050 to 0.008% by mass, or from 0.0050 to 0.007% by mass.

In view of more satisfactorily achieving the effect of the present invention, the porous deodorant may comprise at least one selected from the group consisting of activated carbons, zeolite, silicon dioxide, silicates, titania, alumina, aluminum hydroxide, and magnesium hydroxide, and may comprise at least one selected from the group consisting of zeolite, activated carbons, and silicon dioxide. The porous deodorant preferably comprises at least an activated carbon.

The activated carbon may be produced from, for example, coconut shells, infusibilized or carbonized organic materials, and infusible resins such as phenol resins. Examples of the organic materials include polyacrylonitrile, pitch, polyvinyl alcohol, and cellulose. Among these, the activated carbon is preferably produced from wood (sawdust), coconut shells, or pitch (such as coal pitch).

The porous deodorant may have a median particle size of 1 to 100 $\mu$m, 1 to 80 $\mu$m, 1 to 60 $\mu$m, 10 to 100 $\mu$m, 10 to 80 $\mu$m, 10 to 60 $\mu$m, 15 to 100 $\mu$m, 15 to 80 $\mu$m, 15 to 60 $\mu$m, 20 to 100 $\mu$m, 20 to 80 $\mu$m, or 20 to 60 $\mu$m.

The median particle size (D50 (median size), volume-based) of the porous deodorant can be measured using a laser diffraction particle size distribution analyzer; specifically, it is the value as measured by the method described in the Examples section.

The porous deodorant preferably has a shape such as, for example, a crushed shape or a cylindrical shape, preferably a crushed shape.

The porous deodorant may have a BET specific surface area of 100 to 3000 m$^2$/g, 100 to 2500 m$^2$/g, 100 to 2000 m$^2$/g, 100 to 1500 m$^2$/g, 500 to 3000 m$^2$/g, 500 to 2500 m$^2$/g, 500 to 2000 m$^2$/g, 500 to 1500 m$^2$/g, 1000 to 3000 m$^2$/g, 1000 to 2500 m$^2$/g, 1000 to 2000 m$^2$/g, or 1000 to 1500 m$^2$/g. If the BET specific surface area of the porous deodorant is too large, each pore may be so fine that the strength of the porous deodorant decreases, and the above-described degree of dusting increases. Therefore, the upper limit of the BET specific surface area is preferably 2000 m$^2$/g.

The BET specific surface area of the porous deodorant can be measured using a specific surface area analyzer.

The porous deodorant may have a loss on drying of, for example, 0.1 to 15.0%, 0.1 to 10.0%, 0.1 to 5.0%, 0.5 to 15.0%, 0.5 to 10.0%, 0.5 to 5.0%, 1.0 to 15.0%, 1.0 to 10.0%, or 1.0 to 5.0%.

As used herein, the loss on drying of the porous deodorant is the value as measured according to JIS K 1474: 2014.

In the deodorant composition of the present invention, preferably, the porous deodorant comprising a halogen is disposed on surfaces of the water-absorbent resin particles (i.e., the porous deodorant is present on surfaces of the water-absorbent resin particles). The porous deodorant comprising a halogen can be disposed on the surfaces of the water-absorbent resin particles by, for example, mixing the water-absorbent resin particles and the porous deodorant comprising a halogen in a solid phase, so that the porous deodorant comprising a halogen adheres to the surfaces of the water-absorbent resin particles.

In view of more satisfactorily achieving the effect of the present invention, in the deodorant composition of the present invention, the ratio (Z/(X + Y)) of the content Z (% by mass) of the water-absorbent resin particles to the sum (X + Y) of the content X (% by mass) of the porous deodorant and the content Y (% by mass) of the halogen is preferably 200 or more, more preferably 500 or more, and still more preferably 950 or more, while the ratio (Z/(X + Y)) is preferably 2000 or less, more preferably 1500 or less, and still more preferably 1300 or less. Preferred ranges include from 200 to 2000, from 200 to 1500, from 200 to 1300, from 500 to 2000, from 500 to 1500, from 500 to 1300, from 950 to 2000, from 950 to 1500, or from 950 to 1300.

Furthermore, in view of more satisfactorily achieving the effect of the present invention, in the deodorant composition of the present invention, the sum (X + Y) of the content X (% by mass) of the porous deodorant and the content Y in % by mass (% by mass) of the halogen is preferably 0.01% by mass or more, more preferably 0.05% by mass or more, and still more preferably 0.08% by mass or more, while the sum (X + Y) is preferably 0.30% by mass or less, more preferably 0.20% by mass or less, and still more preferably 0.15% by mass or less. Preferred ranges include from 0.01 to 0.30% by mass, from 0.01 to 0.20% by mass, from 0.01 to 0.15% by mass, from 0.05 to 0.30% by mass, from 0.05 to 0.2% by mass, from 0.05 to 0.15% by mass, from 0.08 to 0.30% by mass, from 0.08 to 0.20% by mass, or from 0.08 to 0.15% by mass.

Furthermore, in view of more satisfactorily achieving the effect of the present invention, the content of the halogen in the porous deodorant comprising a halogen is preferably 0.3% by mass or more, more preferably 1.5% by mass or more, and still more preferably 2.4% by mass or more, while the content of the halogen is preferably 10.0% by mass or less, more preferably 8.0% by mass or less, and still more preferably 7.0% by mass or less. Preferred ranges include from 0.3 to 10% by mass, from 0.3 to 8.0% by mass, from 0.3 to 7.0% by mass, from 1.5 to 10.0% by mass, from 1.5 to 8.0% by mass, from 1.5 to 7.0% by mass, from 2.4 to 10.0% by mass, from 2.4 to 8.0% by mass, or from 2.4 to 7.0% by mass.

The water-absorbent resin particles contained in the deodorant composition of the present invention will be described next in detail.

(Water-Absorbent Resin Particles)

The water-absorbent resin particles contained in the deodorant composition of the present invention are formed of a crosslinked product of a polymer of a water-soluble ethylenically unsaturated monomer, that is, a crosslinked polymer having a structural unit derived from a water-soluble ethylenically unsaturated monomer.

The water-absorbent resin particles preferably have a water absorption rate, as measured by a Vortex method, of preferably 50 seconds or less, more preferably 44 seconds or less, still more preferably 35 seconds or less, particularly preferably 10 seconds or less. On the other hand, the water absorption rate is preferably 1 second or more, and more preferably 2 seconds or more. Preferred ranges include from 1 to 50 seconds, from 1 to 44 seconds, from 1 to 35 seconds, from 1 to 10 seconds, from 2 to 50 seconds, from 2 to 44 seconds, from 2 to 35 seconds, or from 2 to 10 seconds.

The water absorption rate of the water-absorbent resin particles as measured by the Vortex method is the value as measured by the method described in the Examples section.

The water-absorbent resin particles may have a median particle size of, for example, 150 to 850 $\mu$m, 150 to 600 $\mu$m, 150 to 550 $\mu$m, 150 to 500 $\mu$m, 150 to 450 $\mu$m, 150 to 400 $\mu$m, 200 to 850 $\mu$m, 200 to 600 $\mu$m, 200 to 550 $\mu$m, 200 to 500 $\mu$m, 200 to 450 $\mu$m, 200 to 400 $\mu$m, 240 to 850 $\mu$m, 240 to 600 $\mu$m, 240 to 550 $\mu$m, 240 to 500 $\mu$m, 240 to 450 $\mu$m, 240 to 400 $\mu$m, 260 to 850 $\mu$m, 260 to 600 $\mu$m, 260 to 550 $\mu$m, 260 to 500 $\mu$m, 260 to 450 $\mu$m, 260 to 400 $\mu$m, 280 to 850 $\mu$m, 280 to 600 $\mu$m, 280 to 550 $\mu$m, 280 to 500 $\mu$m, 280 to 450 $\mu$m, 280 to 400 $\mu$m, 300 to 850 $\mu$m, 300 to 600 $\mu$m, 300 to 550 $\mu$m, 300 to 500 $\mu$m, 300 to 450 $\mu$m, or 300 to 400 $\mu$m.

The water-absorbent resin particles may be in the form of particles each composed of a single particle, or in the form of particles (secondary particles) formed by agglomeration of fine particles (primary particles). Examples of the shape of the primary particles include a substantially spherical shape, a crushed indefinite shape, and a flat shape. When the primary particles are those produced by reversed phase suspension polymerization, examples of the shape include a substantially spherical single-particle shape with a smooth surface shape, such as a spherical shape or an elliptical sphere shape.

The median particle size of the water-absorbent resin particles can be measured using JIS standard sieves; specifically, it is the value as measured by the method described in the Examples section.

The water-absorbent resin particles have a structural unit derived from a neutralized salt of a water-soluble ethylenically unsaturated monomer. A representative polymerization method, such as aqueous polymerization, emulsion polymerization, or reversed phase suspension polymerization, is used for polymerizing the water-soluble ethylenically unsaturated monomer. In aqueous polymerization, polymerization is performed by heating, optionally with stirring, a water-soluble ethylenically unsaturated monomer solution. In reversed phase suspension polymerization, polymerization is performed by heating the water-soluble ethylenically unsaturated monomer with stirring in a hydrocarbon dispersion medium.

One exemplary method for producing the water-absorbent resin particles will be hereinafter described.

Specific examples of methods for producing the water-absorbent resin particles include a method for producing the water-absorbent resin particles by performing reversed phase suspension polymerization of the water-soluble ethylenically unsaturated monomer in a hydrocarbon dispersion medium, the method including the steps of performing the polymerization in the presence of a radical polymerization initiator; and surface-crosslinking a hydrous gel obtained by the polymerization, in the presence of a surface-crosslinking agent. In the method for producing the water-absorbent resin particles of the present invention, an internal-crosslinking agent may be optionally added to the water-soluble ethylenically unsaturated monomer to obtain a hydrous gel having an internal-crosslinked structure.

<Polymerization Step>

[Water-Soluble Ethylenically Unsaturated Monomer]

Examples of the water-soluble ethylenically unsaturated monomer include (meth)acrylic acid ("acrylic" and "methacrylic" are herein collectively referred to as "(meth)acrylic"; the same applies below) and salts thereof; 2-(meth)acrylamido-2-methylpropanesulfonic acid and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; and amino group-containing unsaturated monomers, such as N,N-diethylaminoethyl (meth)acrylate, N,N-diethylaminopropyl (meth)acrylate, and diethylaminopropyl (meth)acrylamide, as well as quaternary compounds thereof. Preferred among these water-soluble ethylenically unsaturated monomers are (meth)acrylic acid and salts thereof, (meth)acrylamide, and N,N-dimethylacrylamide, and more preferred are (meth)acrylic acid and salts thereof, because they are readily industrially available, for example. These water-soluble ethylenically unsaturated monomers may be used alone or in combinations of two or more.

Among these water-soluble ethylenically unsaturated monomers, acrylic acid and salts thereof are widely used as raw materials of water-absorbent resin particles. Copolymers of acrylic acid and/or salts thereof with other water-soluble ethylenically unsaturated monomers as mentioned above may also be used. In this case, acrylic acid and/or a salt thereof as a main water-soluble ethylenically unsaturated monomer is preferably used in an amount of 70 to 100 mol% based on the total amount of water-soluble ethylenically unsaturated monomers.

The water-soluble ethylenically unsaturated monomer as an aqueous solution may be dispersed in a hydrocarbon dispersion medium and then subjected to reversed phase suspension polymerization. When the water-soluble ethylenically unsaturated monomer is in the form of an aqueous solution, the dispersion efficiency in the hydrocarbon dispersion medium can be increased. The concentration of the water-soluble ethylenically unsaturated monomer in the aqueous solution is preferably in the range of 20% by mass to not more than the saturation concentration. The concentration of the water-soluble ethylenically unsaturated monomer is more preferably 55% by mass or less, still more preferably 50% by mass or less, and even more preferably 45% by mass or less. On the other hand, the concentration of the water-soluble ethylenically unsaturated monomer is more preferably 25% by mass or more, still more preferably 28% by mass or more, and even more preferably 30% by mass or more.

When the water-soluble ethylenically unsaturated monomer has an acid group such as (meth)acrylic acid or 2-(meth)acrylamido-2-methylpropanesulfonic acid, the acid group may be optionally neutralized with an alkaline neutralizing agent, before the water-soluble ethylenically unsaturated monomer is used. Examples of such alkaline neutralizing agents include alkali metal salts, such as sodium hydroxide, sodium carbonate, sodium hydrogen carbonate, potassium hydroxide, and potassium carbonate; and ammonia. These alkaline neutralizing agents may be used in the form of an aqueous solution to facilitate the neutralization process. The above-mentioned alkaline neutralizing agents may be used alone or in combinations of two or more.

The neutralization degree of the water-soluble ethylenically unsaturated monomer with an alkaline neutralizing agent, calculated as the neutralization degree of all acid groups in the water-soluble ethylenically unsaturated monomer, is preferably 10 to 100 mol%, more preferably 30 to 90 mol%, still more preferably 40 to 85 mol%, and even more preferably 50 to 80 mol%.

[Radical Polymerization Initiator]

Examples of the radical polymerization initiator to be added in the polymerization step include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and azo compounds, such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Preferred among these radical polymerization initiators are potassium persulfate, ammonium persulfate, sodium persulfate, and 2,2'-azobis(2-amidinopropane) dihydrochloride, because they are readily available and easy to handle. These radical polymerization initiators may be used alone or in combinations of two or more. The above-mentioned radical polymerization initiators may also be used in combination with a reducing agent, such as sodium sulfite, sodium hydrogensulfite, ferrous sulfate, or L-ascorbic acid, and used as a redox polymerization initiator.

The amount of the radical polymerization initiator used is, for example, 0.00005 to 0.01 mol per mole of the water-soluble ethylenically unsaturated monomer. When the radical polymerization initiator is used in the above-defined range of amounts, the occurrence of an abrupt polymerization reaction can be avoided, and the polymerization reaction can be completed in an appropriate period of time.

[Internal-Crosslinking Agent]

Examples of the internal-crosslinking agent include those that can crosslink the polymer of the water-soluble ethylenically unsaturated monomer to be used, for example: unsaturated polyesters obtained by reacting polyols, such as diols and triols, e.g., (poly)ethylene glycol ["(poly)" means both cases with and without the prefix "poly"; the same applies below], (poly)propylene glycol, 1,4-butanediol, 1,6-hexanediol, trimethylolpropane, and (poly)glycerin, with unsaturated acids, such as (meth)acrylic acid, maleic acid, and fumaric acid; bisacrylamides, such as N,N-methylenebisacrylamide; di or tri(meth)acrylic acid esters obtained by reacting polyepoxides with (meth)acrylic acid; carbamyl di(meth)acrylates obtained by reacting polyisocyanates, such as tolylene diisocyanate and hexamethylene diisocyanate, with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds, such as diglycidyl compounds, e.g., (poly)ethylene glycol diglycidyl ether, (poly)propylene glycol diglycidyl ether, and (poly)glycerin diglycidyl ether, and triglycidyl compounds; epihalohydrin compounds, such as epichlorohydrin, epibromohydrin, and α-methylepichlorohydrin; compounds having two or more reactive functional groups, such as isocyanate compounds, e.g., 2,4-tolylene diisocyanate and hexamethylene diisocyanate; and oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are still more preferably used. These internal-crosslinking agents may be used alone or in combinations of two or more.
The amount of the internal-crosslinking agent used is preferably 0.000001 to 0.02 mol, more preferably 0.00001 to 0.01 mol, still more preferably 0.00001 to 0.005 mol, and even more preferably 0.00005 to 0.002 mol, per mole of the water-soluble ethylenically unsaturated monomer.

[Hydrocarbon Dispersion Medium]

Examples of the hydrocarbon dispersion medium include $C_{6-8}$ aliphatic hydrocarbons, such as n-hexane, n-heptane, 2-methylhexane, 3-methylhexane, 2,3-dimethylpentane, 3-ethylpentane, and n-octane; alicyclic hydrocarbons, such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons, such as benzene, toluene, and xylene. Among these hydrocarbon dispersion media, n-hexane, n-heptane, and cyclohexane, which are readily industrially available, stable in quality, and inexpensive, are particularly suitably used. These hydrocarbon dispersion media may be used alone or in combinations of two or more. The use of a commercially available mixture of hydrocarbon dispersion media, such as Exxsol Heptane (from Exxon Mobil Corporation; containing 75 to 85% by mass of heptane and its isomeric hydrocarbons), also leads to favorable results.
The amount of the hydrocarbon dispersion medium used is preferably 100 to 1500 parts by mass, and more preferably 200 to 1400 parts by mass, per 100 parts by mass of a first-stage water-soluble ethylenically unsaturated monomer, in view of homogeneously dispersing the water-soluble ethylenically unsaturated monomer, and facilitating control of the polymerization temperature. As described below, reversed phase suspension polymerization is performed in one stage (single stage) or two or more multiple stages. The above-mentioned first-stage polymerization refers to the first-stage polymerization reaction in single-stage polymerization or multi-stage polymerization (the same applies below).

[Dispersion Stabilizer]

(Surfactant)

In reversed phase suspension polymerization, a dispersion stabilizer may be used to improve the dispersion stability of the water-soluble ethylenically unsaturated monomer in the hydrocarbon dispersion medium. A surfactant may be used as such a dispersion stabilizer.
Examples of usable surfactants include sucrose fatty acid esters, polyglycerol fatty acid esters, sorbitan fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene-polyoxypropylene block copolymers, polyoxyethylene polyoxypropyl alkyl ethers, polyethylene glycol fatty acid esters, alkyl glucosides, N-alkyl glyconamides, polyoxyethylene fatty acid amides, polyoxyethylene alkylamines, polyoxyethylene alkyl ether phosphates, and polyoxyethylene alkyl allyl ether phosphates. Among these surfactants, sorbitan fatty acid esters, polyglycerol fatty acid esters, and sucrose fatty acid esters are particularly preferably used, in view of dispersion stability of the monomer. These surfactants may be used alone or in combinations of two or more.

The amount of the surfactant used is preferably 0.1 to 30 parts by mass, and more preferably 0.3 to 20 parts by mass, per 100 parts by mass of the first-stage water-soluble ethylenically unsaturated monomer.

(Polymeric Dispersion Agent)

A polymeric dispersion agent may be used in combination with the above-described surfactant, as the dispersion stabilizer to be used in reversed phase suspension polymerization.

Examples of the polymeric dispersion agent include maleic anhydride modified polyethylene, maleic anhydride modified polypropylene, maleic anhydride modified ethylene-propylene copolymers, maleic anhydride modified EPDM (ethylene-propylene-diene terpolymers), maleic anhydride modified polybutadiene, maleic anhydride-ethylene copolymers, maleic anhydride-propylene copolymers, maleic anhydride-ethylene-propylene copolymers, maleic anhydride-butadiene copolymers, polyethylene, polypropylene, ethylene-propylene copolymers, oxidized polyethylene, oxidized polypropylene, oxidized ethylene-propylene copolymers, ethylene-acrylic acid copolymers, ethyl cellulose, and ethyl hydroxyethyl cellulose. Among these polymeric dispersion agents, maleic anhydride modified polyethylene, maleic anhydride modified polypropylene, maleic anhydride modified ethylene-propylene copolymers, maleic anhydride-ethylene copolymers, maleic anhydride-propylene copolymers, maleic anhydride-ethylene-propylene copolymers, polyethylene, polypropylene, ethylene-propylene copolymers, oxidized polyethylene, oxidized polypropylene, and oxidized ethylene-propylene copolymers are particularly preferably used, in view of dispersion stability of the monomer. These polymeric dispersion agents may be used alone or in combinations of two or more.

The amount of the polymeric dispersion agent used is preferably 0.1 to 30 parts by mass, and more preferably 0.3 to 20 parts by mass, per 100 parts by mass of the first-stage water-soluble ethylenically unsaturated monomer.

[Other Components]

In the method for producing the water-absorbent resin particles, other components may be optionally added to the aqueous solution containing the water-soluble ethylenically unsaturated monomer, which is then subjected to reversed phase suspension polymerization. Various additives such as thickeners and chain transfer agents can be added as the other components.

By way of example, a thickener may be added to the aqueous solution containing the water-soluble ethylenically unsaturated monomer, which is then subjected to reversed phase suspension polymerization. By thus adding a thickener to adjust the viscosity of the aqueous solution, the median particle size obtained by reversed phase suspension polymerization can be controlled.

Examples of usable thickeners include hydroxyethylcellulose, hydroxypropylcellulose, methylcellulose, carboxymethylcellulose, polyacrylic acid, (partially) neutralized polyacrylic acid, polyethylene glycol, polyacrylamide, polyethyleneimine, dextrin, sodium alginate, polyvinyl alcohol, polyvinylpyrrolidone, and polyethylene oxide. Assuming that the stirring rate during the polymerization is the same, the higher the viscosity of the water-soluble ethylenically unsaturated monomer solution, the larger the primary particles and/or secondary particles of the resulting particles tend to be.

[Reversed Phase Suspension Polymerization]

Reversed phase suspension polymerization involves dispersing an aqueous monomer solution containing the water-soluble ethylenically unsaturated monomer in a hydrocarbon dispersion medium, in the presence of a dispersion stabilizer, for example. Here, as long as the dispersion stabilizer (a surfactant and a polymeric dispersion agent) is added before the beginning of the polymerization reaction, it may be added either before or after the addition of the aqueous monomer solution.

In particular, in view of readily reducing the amount of the hydrocarbon dispersion medium remaining in the resulting water-absorbent resin particles, it is preferred to disperse the aqueous monomer solution in the hydrocarbon dispersion medium in which a polymeric dispersion agent is dispersed and then disperse a surfactant therein, followed by polymerization.

The polymerization step can be performed in one stage or in two or more multiple stages.

Reversed phase suspension polymerization in two or more multiple stages may be performed as follows: the first stage of reversed phase suspension polymerization is performed; subsequently, the ethylenically unsaturated monomer is added to and mixed with the reaction mixture obtained by the first-stage polymerization reaction, and the second and subsequent stages of reversed phase suspension polymerization are performed in the same manner as in the first stage. In each of the second and subsequent stages of reversed phase suspension polymerization, in addition to the ethylenically unsaturated monomer, the above-described radical polymerization initiator and/or various additives may be added within the above-defined range of ratios of each component relative to the ethylenically unsaturated monomer, based on the amount of the ethylenically unsaturated monomer added in each of the second and subsequent stages of reversed phase suspension polymerization, and then reversed phase suspension polymerization may be performed.

[Step of Adjusting Particle Size]

The method for producing the water-absorbent resin particles of the present invention may include the step of adjusting a particle size. The step of adjusting a particle size can be performed, for example, by adding a flocculating agent such as a powdery inorganic flocculating agent into the system.

Examples of the powdery inorganic flocculating agent include amorphous silica, zeolite, bentonite, aluminum oxide, talc, titanium dioxide, kaolin, clay, and hydrotalcite. Among these, the powdery inorganic flocculating agent may be at least one selected from the group consisting of amorphous silica, aluminum oxide, talc, and kaolin, because of their high flocculating effect.

When the flocculating agent is added into the system, it is added, for example, between the above-described polymerization step and the below-described drying step, and/or during the below-described surface-crosslinking step.

The amount of the flocculating agent added may be 0.001 to 1 part by mass, 0.005 to 0.5 parts by mass, or 0.01 to 0.2 parts by mass, per 100 parts by mass of the water-soluble ethylenically unsaturated monomer.

The reaction temperature during the polymerization reaction is preferably 20 to 110°C, and more preferably 40 to 90°C, in view of allowing the polymerization to proceed quickly to reduce the polymerization time for improved economic efficiency, and readily removing the heat of polymerization to perform the reaction smoothly.

<Surface-Crosslinking Step>

Next, the water-absorbent resin particles of the present invention are obtained by crosslinking a hydrous gel-like polymer having an internal-crosslinked structure obtained by polymerization of the water-soluble ethylenically unsaturated monomer, by adding a surface-crosslinking agent thereto (surface-crosslinking reaction). The surface-crosslinking reaction is preferably performed in the presence of a surface-crosslinking agent, after the polymerization of the water-soluble ethylenically unsaturated monomer. By thus subjecting the hydrous gel-like polymer having an internal-crosslinked structure to the surface-crosslinking reaction after the polymerization, it is possible to obtain water-absorbent resin particles having an increased crosslinking density near the surfaces of the water-absorbent resin particles to exhibit improvement in various kinds of performance, such as water absorption capacity under load.

Examples of the surface-crosslinking agent include compounds with two or more reactive functional groups. Examples include polyols, such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds, such as epichlorohydrin, epibromohydrin, and $\alpha$-methyl epichlorohydrin; isocyanate compounds, such as 2,4-tolylene diisocyanate and hexamethylene diisocyanate; oxetane compounds, such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds, such as 1,2-ethylenebisoxazoline; carbonate compounds (e.g., alkylene carbonates), such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxolan-2-one; and hydroxyalkylamide compounds, such as bis[N,N-di($\beta$-hydroxyethyl)]adipamide. Preferred among these surface-crosslinking agents are polyglycidyl compounds, such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. These surface-crosslinking agents may be used alone or in combinations of two or more.

The amount of the surface-crosslinking agent used is preferably 0.00001 to 0.01 mol, more preferably 0.00005 to 0.005 mol, and still more preferably 0.0001 to 0.002 mol, per mole of the total amount of the water-soluble ethylenically unsaturated monomer used for polymerization.

The surface-crosslinking agent may be added as is or as an aqueous solution. Optionally, a solution of the surface-crosslinking agent in a hydrophilic organic solvent may be added. Examples of the hydrophilic organic solvent include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; and sulfoxides, such as dimethylsulfoxide. These hydrophilic organic solvents may be used alone, in combinations of two or more, or as a solvent mixture with water.

The surface-crosslinking agent may be added at any time after the polymerization reaction of the water-soluble ethylenically unsaturated monomer is substantially completed. The surface-crosslinking agent is preferably added in the presence of 1 to 400 parts by mass of water, more preferably 5 to 200 parts by mass of water, still more preferably 10 to 100 parts by mass of water, even more preferably 20 to 60 parts by mass of water, per 100 parts by mass of the water-soluble ethylenically unsaturated monomer. The amount of water herein refers to the total amount of the water contained in the reaction system and the water optionally used during the addition of the surface-crosslinking agent.

The reaction temperature during the surface-crosslinking reaction is preferably 50 to 250°C, more preferably 60 to 180°C, still more preferably 60 to 140°C, and even more preferably 70 to 120°C. The reaction time of the surface-crosslinking reaction is preferably 1 to 300 minutes, and more preferably 5 to 200 minutes.

<Drying Step>

After reversed phase suspension polymerization is performed as described above, the method may include a drying step of removing the water, the hydrocarbon dispersion medium, and the like by distillation, by adding external energy, such as heat. To remove the water in the hydrous gel-like polymer after reversed phase suspension polymerization, the system in which the hydrous gel-like polymer is dispersed in the hydrocarbon dispersion medium is heated to distill the water and the hydrocarbon dispersion medium out of the system by azeotropic distillation. Here, if the distilled hydrocarbon dispersion medium only is returned into the system, continuous azeotropic distillation can be performed. This is preferable in view of preventing deterioration of the resin, because the temperature within the system during drying is maintained at a temperature not higher than the azeotropic temperature with the hydrocarbon dispersion medium. Subsequently, the water and the hydrocarbon dispersion medium are distilled off to obtain water-absorbent resin particles. By controlling the treatment conditions for the drying step after the polymerization to adjust the amount of water to be removed, various kinds of performance of the resulting water-absorbent resin particles can be controlled.

In the drying step, the drying treatment by distillation may be performed under atmospheric pressure or reduced pressure. The drying treatment may also be performed in a stream of nitrogen or the like, in view of improving the drying efficiency. When the drying treatment is performed under atmospheric pressure, the drying temperature is preferably 70 to 250°C, more preferably 80 to 180°C, still more preferably 80 to 140°C, and even more preferably 90 to 130°C. When the drying treatment is performed under reduced pressure, the drying temperature is preferably 40 to 160°C, and more preferably 50 to 110°C.

When the surface-crosslinking step with a surface-crosslinking agent is performed after the polymerization of the monomer by reversed phase suspension polymerization, the drying step by distillation is performed as described above, after the completion of the surface-crosslinking step. Alternatively, the surface-crosslinking step and the drying step may be performed simultaneously.

The deodorant composition of the present invention may contain additives suitable for its purpose. Examples of such additives include inorganic powders, surfactants, oxidizing agents, reducing agents, metal chelating agents, radical chain inhibitors, antioxidants, and anti-bacterial agents. For example, when 0.05 to 5 parts by mass of amorphous silica as an inorganic powder is added to 100 parts by mass of the water-absorbent resin particles, the flowability of the deodorant composition can be further improved. The additives are preferably hydrophilic or water-soluble.

Furthermore, in view of more satisfactorily achieving the effect of the present invention, in the deodorant composition of the present invention, the content Z (% by mass) of the water-absorbent resin particles is preferably 95.00% by mass or more, more preferably 98.00% by mass or more, and still more preferably 99.00% by mass or more, while the content Z is preferably 99.99% by mass or less, more preferably 99.98% by mass or less, and still more preferably 99.97% by mass or less. A preferred range may be, for example, from 95.00 to 99.99% by mass.

The deodorant composition of the present invention can be produced, for example, by mixing the water-absorbent resin particles and the porous deodorant comprising a halogen in a solid phase.

The deodorant composition of the present invention can be used for a wide range of deodorizing applications, and is particularly suitable for deodorizing applications requiring water absorbency because it contains the water-absorbent resin particles. As described later, the deodorant composition of the present invention can be suitably used for an absorbent material used for hygienic materials, such as disposable diapers, for example.

2. Absorbent Material and Absorbent Article

The deodorant composition of the present invention can be suitably used for an absorbent material used for hygienic materials, such as disposable diapers, for example. When the content Z of the water-absorbent resin particles in the deodorant composition of the present invention is high (for example, when the content Z (% by mass) of the water-absorbent resin particles in the deodorant composition of the present invention is 95.00% by mass or more), the deodorant composition of the present invention can be used as a water-absorbent resin composition for the absorbent material. The absorbent material is suitably used for an absorbent article including the absorbent material. In this case, the absorbent article of the present invention includes the deodorant composition of the present invention.

Herein, the absorbent material obtained using the deodorant composition of the present invention contains the deodorant composition of the present invention. The absorbent material may further contain hydrophilic fibers. Examples of the structure of the absorbent material include a sheet-like structure in which the water-absorbent resin particles are fixed on a nonwoven fabric or between a plurality of nonwoven fabrics; a mixed dispersion obtained by mixing the deodorant composition and hydrophilic fibers to give a homogeneous composition; a sandwich structure in which the deodorant composition is sandwiched between layered hydrophilic fibers; and a structure in which the deodorant composition and hydrophilic fibers are wrapped in a tissue. The absorbent material may also contain other components, for example, adhesive binders such as thermal bonding synthetic fibers, hot melt adhesives, and adhesive emulsions, for improving the shape retention properties of the absorbent material.

The content of the deodorant composition in the absorbent material is preferably 5 to 100% by mass, more preferably 10 to 95% by mass, still more preferably 20 to 90% by mass, and even more preferably 30 to 80% by mass.

Examples of the hydrophilic fibers include cellulose fibers, such as cotton-like pulp made from wood, mechanical pulp, chemical pulp, and semi-chemical pulp; artificial cellulose fibers, such as rayon and acetate; and fibers made of synthetic resins, such as hydrophilized polyamides, polyesters, and polyolefins. The hydrophilic fibers typically have an average fiber length of 0.1 to 10 mm. Alternatively, the average fiber length may be 0.5 to 5 mm.

It is possible to obtain the absorbent article of the present invention by holding the absorbent material obtained using the deodorant composition of the present invention between a liquid-permeable sheet (top sheet) that allows a liquid to pass through and a liquid-impermeable sheet (back sheet) that does not allow a liquid to pass through. The liquid-permeable sheet is positioned on the side that is brought into contact with the body, and the liquid-impermeable sheet is positioned opposite to the side that is brought into contact with the body.

Examples of the liquid-permeable sheet include porous synthetic resin sheets and nonwoven fabrics of the types such as air-through type, spunbond type, chemical bond type, and needle punch type, which are made of fibers of polyethylene, polypropylene, polyester, and the like. Examples of the liquid-impermeable sheet include synthetic resin films made of resins such as polyethylene, polypropylene, and polyvinyl chloride.

3. Additional Matters

The present specification includes at least the inventions as set forth in (1) to (8) below.

(1) A deodorant composition comprising a porous deodorant comprising a halogen; and water-absorbent resin particles, wherein a content of the halogen is 0.0030% by mass or more.

(2) The deodorant composition according to (1) above, wherein the content of the halogen is 0.0030 to 0.010% by mass, 0.0030 to 0.008% by mass, 0.0030 to 0.007% by mass, 0.0035 to 0.010% by mass, 0.0035 to 0.008% by mass, 0.0035 to 0.007% by mass, 0.0040 to 0.010% by mass, 0.0040 to 0.008% by mass, 0.0040 to 0.007% by mass, 0.0050 to 0.010% by mass, 0.0050 to 0.008% by mass, or 0.0050 to 0.007% by mass.

(3) The deodorant composition according to (1) or (2) above, wherein the halogen comprises at least one selected from the group consisting of bromine and chlorine.

(4) The deodorant composition according to any one of (1) to (3) above, wherein the porous deodorant comprises at least one compound selected from the group consisting of activated carbons, silicon dioxide, and silicates.

(5) The deodorant composition according to any one of (1) to (4) above, wherein a ratio $(Z/(X + Y))$ of a content $Z$ (% by mass) of the water-absorbent resin particles to a sum $(X + Y)$ of a content $X$ (% by mass) of the porous deodorant and the content $Y$ (% by mass) of the halogen is 200 to 2000, 200 to 1500, 200 to 1300, 500 to 2000, 500 to 1500, 500 to 1300, 950 to 2000, 950 to 1500, or 950 to 1300.

(6) The deodorant composition according to any one of (1) to (5) above, wherein the sum $(X + Y)$ of the content $X$ (% by mass) of the porous deodorant and the content $Y$ in % by mass (% by mass) of the halogen is 0.01 to 0.30% by mass, 0.01 to 0.20% by mass, 0.01 to 0.15% by mass, 0.05 to 0.30% by mass, 0.05 to 0.2% by mass, 0.05 to 0.15% by mass, 0.08 to 0.30% by mass, 0.08 to 0.20% by mass, or 0.08 to 0.15% by mass.

(7) The deodorant composition according to any one of (1) to (6) above, wherein the water-absorbent resin particles have a water absorption rate, as measured by a Vortex method, of 1 to 50 seconds, 1 to 44 seconds, 1 to 35 seconds, 1 to 10 seconds, 2 to 50 seconds, 2 to 44 seconds, 2 to 35 seconds, or 2 to 10 seconds.

(8) The deodorant composition according to any one of (1) to (7) above, wherein the porous deodorant comprises a halogen, and a content of the halogen in the porous deodorant comprising the halogen is 0.3 to 10.0% by mass, 0.3 to 8.0% by mass, 0.3 to 7.0% by mass, 1.5 to 10.0% by mass, 1.5 to 8.0% by mass, 1.5 to 7.0% by mass, 2.4 to 10.0% by mass, 2.4 to 8.0% by mass, or 2.4 to 7.0% by mass.

Examples

The present invention will be hereinafter described in detail with reference to examples and comparative examples. However, the present invention is not limited to the examples.

The below-described water-absorbent resin particles, activated carbons serving as porous deodorants, and deodorant compositions obtained in the examples and comparative examples were evaluated using the tests as described below. Unless otherwise indicated, the measurement was performed in an environment at a temperature of $25 \pm 2°C$ and a humidity of $50 \pm 10\%$.

[Production Examples of Water-Absorbent Resin Particles]

<Production Example 1>

A 110 mm inner diameter, 2 L volume, cylindrical round-bottomed separable flask with four side wall baffles (baffle width: 7 mm) was prepared which was equipped with a reflux condenser, a dropping funnel, a nitrogen gas inlet tube, and an agitator. The agitator was equipped with stirring blades having two stages of four inclined paddle blades surface-treated with a fluororesin and having a blade diameter of 50 mm. In the prepared separable flask, 451.4 g of n-heptane and 1.288 g of sorbitan monolaurate (trade name: NONION LP-20R, HLB value: 8.6, from NOF CORPORATION) were mixed. The mixture in the separable flask was heated to 50°C while stirring with the agitator to dissolve the sorbitan monolaurate in the n-heptane. The resulting solution was cooled to 40°C.

92.0 g of an 80.5% by mass aqueous solution of acrylic acid (acrylic acid: 1.03 mol) was placed in a 500 mL inner volume Erlenmeyer flask. 147.7 g of a 20.9% by mass aqueous solution of sodium hydroxide was added dropwise into the aqueous solution of acrylic acid in the flask, with external ice-cooling, to partially neutralize the acrylic acid. Subsequently, 0.1011 g (0.374 mmol) of potassium persulfate was added as a water-soluble radical polymerization initiator and dissolved in the aqueous solution to prepare an aqueous solution.

The resulting aqueous solution was added to the separable flask containing the solution containing the sorbitan monolaurate, and the system was sufficiently purged with nitrogen. While stirring with the agitator at a rotation speed of 700 rpm, the reaction mixture in the separable flask was maintained in a warm water bath at 70°C for 60 minutes, causing the polymerization reaction to proceed.

To the reaction mixture containing the hydrous gel-like polymer produced by the polymerization reaction, a dispersion of 0.092 g of amorphous silica (Tokusil NP-S from Oriental Silicas Corporation) in 100 g of n-heptane was added, and the reaction mixture was stirred for 10 minutes. The separable flask was immersed in an oil bath at 125°C to remove 98.5 g of water out of the system by azeotropic distillation. Then, 4.14 g of a 2% by mass aqueous solution of ethylene glycol diglycidyl ether (ethylene glycol diglycidyl ether: 0.475 mmol) was added as a surface-crosslinking agent, and the flask was maintained at an internal temperature of $80 \pm 2°C$ for 2 hours to cause the surface-crosslinking reaction to proceed.

The reaction mixture was heated to 125°C to evaporate the water and n-heptane, thus giving a dried product of water-absorbent resin particles. This dried product was passed through a sieve with a mesh size of 850 μm to give 86.3 g of water-absorbent resin particles. The water-absorbent resin particles had a physiological saline retention capacity of 37 g/g, a water absorption rate of 3 seconds, a median particle size of 361 μm, and a physiological saline absorption capacity under a load of 4.14 kPa of 11 ml/g.

<Production Example 2>

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.092 g of hydroxyethyl cellulose (HEC AW-15F from Sumitomo Seika Chemicals Co., Ltd.) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 550 rpm. Then, the flask was immersed in a water bath at 70°C and heated, and polymerization was performed for 60 minutes to give a first-stage polymerization slurry.

Separately, in another 500 mL inner volume beaker, 128.8 g (1.43 mol) of an 80.5% by mass aqueous solution of acrylic acid was placed as a water-soluble ethylenically unsaturated monomer, and 159.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0117 g (0.067 mmol) of ethylene glycol diglycidyl ether as an internal-crosslinking agent were added and dissolved to prepare a second-stage aqueous solution.

The separable flask system was cooled to 27°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give a hydrous gel-like polymer.

Then, the flask was immersed in an oil bath set at 125°C to remove 259.9 g of water out of the system by azeotropic distillation of the water and n-heptane, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours.

Then, the reaction mixture was dried by evaporating the n-heptane at 125°C, and the resulting product was subsequently passed through a sieve with a mesh size of 850 μm to give 220.4 g of water-absorbent resin particles. The water-absorbent resin particles had a physiological saline retention capacity of 40 g/g, a water absorption rate of 32 seconds, a median particle size of 278 μm, and a physiological saline absorption capacity under a load of 4.14 kPa of 20 ml/g.

<Production Example 3>

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.092 g of hydroxyethyl cellulose (HEC AW-15F from Sumitomo Seika Chemicals Co., Ltd.) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 550 rpm. Then, the flask was immersed in a water bath at 70°C and heated, and polymerization was performed for 60 minutes to give a first-stage polymerization slurry.

Separately, in another 500 mL inner volume beaker, 128.8 g (1.43 mol) of an 80.5% by mass aqueous solution of acrylic acid was placed as a water-soluble ethylenically unsaturated monomer, and 159.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0117 g (0.067 mmol) of ethylene glycol diglycidyl ether as an internal-crosslinking agent were added and dissolved to prepare a second-stage aqueous solution.

The separable flask system was cooled to 25°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give a hydrous gel-like polymer.

Then, the flask was immersed in an oil bath set at 125°C to remove 259.9 g of water out of the system by azeotropic distillation of the water and n-heptane, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours.

Then, the reaction mixture was dried by evaporating the n-heptane at 125°C, and the resulting product was subsequently passed through a sieve with a mesh size of 850 μm to give 226.1 g of water-absorbent resin particles. The water-absorbent resin particles had a physiological saline retention capacity of 42 g/g, a water absorption rate of 42 seconds, a median particle size of 366 μm, and a physiological saline absorption capacity under a load of 4.14 kPa of 20 ml/g.

<Production Example 4>

An 11 cm inner diameter, 2 L volume, cylindrical round-bottomed separable flask was prepared which was equipped with a reflux condenser, a dropping funnel, a nitrogen gas inlet tube, and an agitator with stirring blades having two stages of four inclined paddle blades with a blade diameter of 5 cm. As a hydrocarbon dispersion medium, 293 g of n-heptane was placed in this flask, and 0.736 g of a maleic anhydride-modified ethylene-propylene copolymer (Hi-Wax 1105A from Mitsui Chemicals, Inc.) was added as a polymeric dispersion agent. The mixture was heated with stirring to 80°C to dissolve the dispersion agent and then cooled to 50°C. Separately, in a 300 mL inner volume beaker, 92.0 g (1.03 mol) of an 80.5% by mass aqueous solution of acrylic acid was placed as a water-soluble ethylenically unsaturated monomer, and 147.7 g of a 20.9% by mass aqueous solution of sodium hydroxide was added dropwise, while cooling with ice water, to achieve 75 mol% neutralization. Then, 0.092 g of hydroxyethyl cellulose (HEC AW-15F from Sumitomo Seika Chemicals Co., Ltd.) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added and dissolved to prepare a first-stage aqueous solution. Then, the aqueous solution prepared as above was added to the separable flask and stirred for 10 minutes. Then, a surfactant solution prepared in a 20 mL vial by heating and dissolving 0.736 g of sucrose stearate having an HLB of 3 (Ryoto sugar ester S-370 from Mitsubishi-Kagaku Foods Corporation) as a surfactant in 6.62 g of n-heptane was further added. The system was sufficiently purged with nitrogen while stirring with the agitator at a rotation speed of 550 rpm. Then, the flask was immersed in a water bath at 70°C and heated, and polymerization was performed for 60 minutes to give a first-stage polymerization slurry.

Separately, in another 500 mL inner volume beaker, 128.8 g (1.43 mol) of an 80.5% by mass aqueous solution of acrylic acid was placed as a water-soluble ethylenically unsaturated monomer, and 159.0 g of a 27% by mass aqueous solution of sodium hydroxide was added dropwise, while cooling with ice water, to achieve 75 mol% neutralization. Then, 0.103 g (0.381 mmol) of potassium persulfate as a water-soluble radical polymerization initiator and 0.0117 g (0.067 mmol) of ethylene glycol diglycidyl ether as an internal-crosslinking agent were added and dissolved to prepare a second-stage aqueous solution.

The separable flask system was cooled to 23°C while stirring with the agitator at a rotation speed of 1000 rpm. Then, the entire amount of the second-stage aqueous solution was added to the first-stage polymerization slurry, and the system was purged with nitrogen for 30 minutes. Then, the flask was again immersed in a water bath at 70°C and heated, and the polymerization reaction was performed for 60 minutes to give a hydrous gel-like polymer.

Then, the flask was immersed in an oil bath set at 125°C to remove 259.9 g of water out of the system by azeotropic distillation of the water and n-heptane, while refluxing the n-heptane. Then, 4.42 g (0.507 mmol) of a 2% by mass aqueous solution of ethylene glycol diglycidyl ether was added to the flask as a surface-crosslinking agent, and the flask was maintained at 83°C for 2 hours.

Then, the reaction mixture was dried by evaporating the n-heptane at 125°C, and the resulting product was subsequently passed through a sieve with a mesh size of 850 μm to give 226.1 g of water-absorbent resin particles. The water-absorbent resin particles had a physiological saline retention capacity of 42 g/g, a water absorption rate of 45 seconds, a median particle size of 389 μm, and a physiological saline absorption capacity under a load of 4.14 kPa of 20 ml/g.

[Evaluation of Water-Absorbent Resin Particles]

<Physiological Saline Absorption Capacity Under a Load of 4.14 kPa>

The physiological saline absorption capacity under a load of 4.14 kPa (water absorption capacity under load) was measured using the measurement apparatus as schematically shown in Fig. 1. Two measurements were made for one type of water-absorbent resin particles, and the average value was obtained. The measurement apparatus includes a burette unit 1, a clamp 3, a conduit 5, a stand 11, a measurement table 13, and a measurement unit 4 placed on the measurement table 13. The burette unit 1 has a graduated burette tube 21, a rubber stopper 23 for sealing the upper opening of the burette tube 21, a cock 22 connected to the lower end of the burette tube 21, an air inlet tube 25 connected to a lower portion of the burette tube 21, and a cock 24. The burette unit 1 is fixed by the clamp 3. The flat measurement table 13 has a through-hole 13a with a diameter of 2 mm formed in the central portion thereof, and is supported by the variable-height stand 11. The through-hole 13a of the measurement table 13 and the cock 22 of the burette unit 1 are connected through the conduit 5. The conduit 5 has an inner diameter of 6 mm.

The measurement unit 4 includes a cylinder 31 made of plexiglas, a polyamide mesh 32 bonded to one opening of the cylinder 31, and a weight 33 vertically movable within the cylinder 31. The cylinder 31 is placed on the measurement table 13 with the polyamide mesh 32 therebetween. The cylinder 31 has an inner diameter of 20 mm. The polyamide mesh 32 has a mesh size of 75 μm (200 mesh). The weight 33 has a diameter of 19 mm and a mass of 119.6 g and, as described later, can apply a load of 4.14 kPa (0.6 psi) to water-absorbent resin particles 10a uniformly disposed on the polyamide mesh 32.

First, the cock 22 and the cock 24 of the burette unit 1 were closed, and 0.9% by mass physiological saline adjusted to 25°C was placed in the burette tube 21 through the upper opening of the burette tube 21. Subsequently, the upper opening of the burette tube 21 was sealed with the rubber stopper 23, and then the cock 22 and the cock 24 were opened. The conduit 5 was filled with the 0.9% by mass saline 50 to prevent air bubbles from entering. The height of the measurement table 13 was adjusted so that the height of the surface of the 0.9% by mass saline reached into the through-hole 13a was equal to the height of the upper surface of the measurement table 13. After the adjustment, the height of the surface of the 0.9% by mass saline 50 in the burette tube 21 was read on the graduations of the burette tube 21, and this position was determined as a zero point (reading at 0 seconds).

In the measurement unit 4, 0.10 g of the water-absorbent resin particles 10a were uniformly disposed on the polyamide mesh 32 in the cylinder 31, the weight 33 was disposed on the water-absorbent resin particles 10a, and the cylinder 31 was placed such that the central portion thereof aligned with the conduit opening at the central portion of the measurement table 13. An amount of reduction Wc (ml) in the physiological saline in the burette tube 21 at 60 minutes after the beginning of absorption of the physiological saline by the water-absorbent resin particles 10a through the conduit 5 (that is, the amount of the physiological saline absorbed by the water-absorbent resin particles 10a) was read, and the physiological saline absorption capacity under a load of 4.14 kPa of the water-absorbent resin particles 10a was calculated using the following equation:

Physiological saline absorption capacity (ml/g) under a load of 4.14 kPa = Wc (ml)/mass (g) of the water-absorbent resin particles

<Measurement of Water Absorption Rate by the Vortex Method>

$50 \pm 0.1$ g of physiological saline adjusted to a temperature of $25 \pm 0.2$°C in a thermostat was weighed into a 100 ml beaker and stirred with a magnetic stirrer bar (8 mm $\phi \times$ 30 mm, no ring) to form a vortex at a rotation speed of 600 rpm. $2.0 \pm 0.002$ g of the water-absorbent resin particles were added at once into the physiological saline, and the time (seconds) from the addition of the water-absorbent resin particles to the point at which the vortex on the liquid surface disappeared was measured. The measured time was determined as the water absorption rate of the water-absorbent resin particles. This water absorption rate is also referred to as the Vortex method or Vortex time.

<Median Particle Size (Particle Size Distribution) of Water-Absorbent Resin Particles>

50.0 g of the water-absorbent resin particles were used for median particle size (particle size distribution) measurement. JIS standard sieves having mesh sizes of 850 $\mu$m, 500 $\mu$m, 425 $\mu$m, 300 $\mu$m, 250 $\mu$m, 180 $\mu$m, and 150 $\mu$m, and a receiving tray were combined in that order from the top. The water-absorbent resin particles were placed on the top sieve of the combined sieves, and classified by shaking for 20 minutes using a Ro-Tap shaker. After the classification, the particle size distribution was determined by calculating the mass of the water-absorbent resin particles remaining on each sieve as the mass percentage relative to the total mass. With regard to this particle size distribution, the mass percentage of the water-absorbent resin particles remaining on each sieve was integrated in descending order of particle size. In this manner, the relationship between the sieve mesh size and the cumulative value of the mass percentage of the water-absorbent resin particles remaining on each sieve was plotted on logarithmic probability paper. The plots on the probability paper were connected with straight lines, and a particle size equivalent to a 50% by mass cumulative mass percentage was determined as the median particle size.

[Preparation of Porous Deodorant]

The following activated carbons A and B were prepared as porous deodorants: activated carbon A was an activated carbon containing bromine molecules as a halogen, and activated carbon B was an activated carbon not containing halogen.

<Activated Carbon A>

The activated carbon (GS2x4/6S) from Osaka Gas Chemicals Co., Ltd. was crushed in a mortar and passed through a sieve with a mesh size of 83 $\mu$m to give activated carbon A. The activated carbon had a median particle size of 48 $\mu$m and a bromine content of 6%.

<Activated Carbon B>

The activated carbon (GH2x4/6S) from Osaka Gas Chemicals Co., Ltd. was crushed in a mortar and passed through a sieve with a mesh size of 83 $\mu$m to give activated carbon B. The activated carbon had a median particle size of 56 $\mu$m and a bromine content of 0%.

[Evaluation of Porous Deodorant]

<Median Particle Size (Laser Diffraction) of Activated Carbon>

The median particle size (D50 (median size), volume-based) of the activated carbon used was measured using a laser diffraction particle size distribution analyzer (SALD-2300 from Shimadzu Corporation).

<Measurement of Bromine Content in Activated Carbon>

A combustion aid was added to 0.5 mg of the activated carbon, and the activated carbon was combusted. The generated gas was collected with 10 mL of an absorption liquid. This absorption liquid was mixed with 5 mL of ultrapure water, and the resulting sample was measured using Thermo Fisher Scientific, ICS-5000. A sample having a high bromine concentration was diluted with ultrapure water and then measured.

[Production of Deodorant Composition]

<Example 1>

Activated carbon A was added in an amount of 0.1 parts by mass to 100 parts by mass of the water-absorbent resin particles of Production Example 1, and the mixture was mixed for 30 minutes using a cross-rotary mixer from Meiwa Kogyo Co., Ltd. (conditions: revolution speed 50 rpm, rotation speed 50 rpm), to give a deodorant composition. The below-described deodorization test was performed using 6 g of the deodorant composition. As a result, the concentration of methyl mercaptan was 9 ppm.

<Example 2>

A deodorant composition was obtained as in Example 1, except that the water-absorbent resin particles of Production Example 2 were used. The below-described deodorization test was performed using 6 g of the deodorant composition. As a result, the concentration of methyl mercaptan was 10 ppm.

<Example 3>

A deodorant composition was obtained as in Example 1, except that the water-absorbent resin particles of Production Example 3 were used. The below-described deodorization test was performed using 6 g of the deodorant composition. As a result, the concentration of methyl mercaptan was 12 ppm.

<Example 4>

A deodorant composition was obtained as in Example 1, except that the water-absorbent resin particles of Production Example 4 were used. The below-described deodorization test was performed using 6 g of the deodorant composition. As a result, the concentration of methyl mercaptan was 13 ppm.

<Example 5>

A deodorant composition was obtained as in Example 1, except that 0.2 parts by mass of activated carbon A was added to 100 parts by mass of the water-absorbent resin particles of Production Example 2. The below-described deodorization test was performed using 3 g of the deodorant composition. As a result, the concentration of methyl mercaptan was 12 ppm.

<Comparative Example 1>

A deodorant composition was obtained as in Example 1, except that the activated carbon to be added to the water-absorbent resin particles was changed to activated carbon B. The below-described deodorization test was performed using 6 g of the deodorant composition. As a result, the concentration of methyl mercaptan was 15 ppm.

<Comparative Example 2>

A deodorant composition was obtained as in Example 4, except that the activated carbon to be added to the water-absorbent resin particles was changed to activated carbon B. The below-described deodorization test was performed using 6 g of the deodorant composition. As a result, the concentration of methyl mercaptan was 15 ppm.

<Comparative Example 3>

The below-described deodorization test was performed using 0.006 mg of activated carbon A alone without using the water-absorbent resin particles. As a result, the concentration of methyl mercaptan was 17 ppm.

<Comparative Example 4>

The below-described deodorization test was performed using 0.006 mg of activated carbon B alone without using the water-absorbent resin particles. As a result, the concentration of methyl mercaptan was 15 ppm.

<Comparative Example 5>

The below-described deodorization test was performed using 6 g of the water-absorbent resin particles of Production Example 1 alone without using any activated carbon. As a result, the concentration of methyl mercaptan was 17 ppm.

<Comparative Example 6>

A petri dish containing artificial urine alone was placed in a sampling bag, and the deodorization test was performed in this state, without using the water-absorbent resin particles and any activated carbon. As a result, the concentration of methyl mercaptan was 17 ppm.

<Reference Example 1>

A petri dish alone was placed in a sampling bag, and the deodorization test was performed in this state, without using the water-absorbent resin particles, any activated carbon, and artificial urine. As a result, the concentration of methyl mercaptan was 20 ppm.

[Deodorization Test]

Artificial urine was prepared by adding 20.0 g of urea, 8.0 g of sodium chloride, 0.3 g of calcium chloride dihydrate, and 0.8 g of magnesium sulfate heptahydrate to 970.0 g of distilled water. A predetermined amount of the deodorant composition was placed in a sterile plastic petri dish with an inner diameter of 70 mm, and 20 mL of the artificial urine was added. The plastic petri dish was sealed in a 2 L sampling bag made of polyester (PAAAK2 from GL Sciences Inc.), the air in the bag was removed using a syringe, and then the bag was allowed to stand for 3 hours. After the completion of 3 hours of standing, 900 mL of air containing methyl mercaptan at a concentration of 20 ppm was sealed in the Smart Bag, and the bag was allowed to stand for 1 hour. After 1 hour, the concentration of methyl mercaptan in the gas phase portion in the Smart Bag was measured using a No. 71 detecting tube (GL Sciences Inc.).

[Table 1]

| | Water-Absorbent Resin Particles | | Porous Deodorant | Halogen | Z/(X+Y) | Deodorization Test Methyl Mercaptan Concentration (ppm) |
| | Water Absorption Rate [sec] | (Z) [% by mass] | (X) [% by mass] | (Y) [% by mass] | | |
|---|---|---|---|---|---|---|
| EX. 1 | 3 | 99.89 | 0.100 | 0.006 | 943 | 9 |

(continued)

| | Water-Absorbent Resin Particles | | Porous Deodorant | Halogen | Z/(X+Y) | Deodorization Test Methyl Mercaptan Concentration (ppm) |
|---|---|---|---|---|---|---|
| | Water Absorption Rate [sec] | (Z) [% by mass] | (X) [% by mass] | (Y) [% by mass] | | |
| Comp.Ex. 1 | 3 | 99.90 | 0.100 | 0 | - | 15 |
| EX. 2 | 32 | 99.89 | 0.100 | 0.006 | 943 | 10 |
| Comp.Ex. 2 | 32 | 99.90 | 0.100 | 0 | 1000 | 15 |
| EX. 3 | 42 | 99.89 | 0.100 | 0.006 | 943 | 12 |
| Comp.Ex. 3 | 42 | 99.90 | 0.100 | 0 | 1000 | 17 |
| EX. 4 | 45 | 99.89 | 0.100 | 0.006 | 943 | 13 |
| Comp.Ex. 4 | 45 | 99.90 | 0.100 | 0 | - | 15 |
| Comp.Ex. 5 | - | 0 | 94.340 | 5.660 | - | 17 |
| Comp.Ex. 6 | - | 0 | 100 | 0 | - | 17 |
| EX. 5 | 32 | 99.79 | 0.200 | 0.012 | 472 | 12 |
| Reference Ex. 1 | - | 0 | 0 | 0 | - | 20 |

As shown in Table 1, each of the deodorant compositions of Examples 1 to 5 comprises a porous deodorant comprising a halogen; and water-absorbent resin particles, wherein a content of the halogen is 0.0030% by mass or more. It can be observed that the deodorant compositions of Examples 1 to 5 have an excellent deodorizing effect against methyl mercaptan, in the deodorization test in an environment where the artificial urine containing polyvalent metal salts and methyl mercaptan coexist. These results indicate that the polyvalent metal salts contained in urine are rapidly absorbed by the water-absorbent resin particles, which prevents the reaction between the halogen molecules in the porous deodorant and the polyvalent metal salts in urine, so that the porous deodorant comprising a halogen exhibits a high deodorizing effect against methyl mercaptan contained in large amounts in feces.

Reference Signs List

1: burette unit
3: clamp
4: measurement unit
5: conduit
10a: water-absorbent resin particles
11: stand
13: measurement table
13a: through-hole
21: burette tube
22: cock
23: rubber stopper
24: cock
25: air inlet tube
31: cylinder
32: polyamide mesh
33: weight
50: saline

**Claims**

1.  A deodorant composition comprising:

    a porous deodorant comprising a halogen; and
    water-absorbent resin particles,
    wherein a content of the halogen is 0.0030% by mass or more.

2.  The deodorant composition according to claim 1, wherein the halogen comprises at least one selected from the group consisting of bromine and chlorine.

3.  The deodorant composition according to claim 1 or 2, wherein the porous deodorant comprises at least one compound selected from the group consisting of activated carbons, zeolite, silicon dioxide, and silicates.

4.  The deodorant composition according to claim 1 or 2, wherein a ratio $(Z/(X + Y))$ of a content Z (% by mass) of the water-absorbent resin particles to a sum $(X + Y)$ of a content X (% by mass) of the porous deodorant and the content Y (% by mass) of the halogen is 200 or more.

5.  The deodorant composition according to claim 1 or 2, wherein the water-absorbent resin particles have a water absorption rate of 50 seconds or less, as measured by a Vortex method.

6.  An absorbent article comprising the deodorant composition according to claim 1 or 2.

FIG. 1

## INTERNATIONAL SEARCH REPORT

| | International application No. |
|---|---|
| | **PCT/JP2024/022474** |

**A. CLASSIFICATION OF SUBJECT MATTER**

*A61F 13/15*(2006.01)i; *A61F 13/53*(2006.01)i
FI: A61F13/15 141; A61F13/53 300

According to International Patent Classification (IPC) or to both national classification and IPC

**B. FIELDS SEARCHED**

Minimum documentation searched (classification system followed by classification symbols)

A61L9/00-9/22; A61F13/15; A61F13/53

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 02-307528 A (DAI NIPPON PRINTING CO., LTD.) 20 December 1990 (1990-12-20) p. 2, lower right column, line 15 to p. 5, lower right column, line 1 | 1-6 |
| Y | JP 2006-022218 A (KOMA, Hiroki) 26 January 2006 (2006-01-26) paragraphs [0013]-[0051] | 1-6 |
| Y | JP 2004-337310 A (CATALER CORP.) 02 December 2004 (2004-12-02) paragraphs [0008]-[0038] | 1-6 |
| Y | JP 2006-061885 A (CATALER CORP.) 09 March 2006 (2006-03-09) paragraphs [0002]-[0025] | 1-6 |

☐ Further documents are listed in the continuation of Box C.       ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **12 July 2024** | **23 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/022474**

| Patent document cited in search report | | | Publication date (day/month/year) | Patent family member(s) | Publication date (day/month/year) |
|---|---|---|---|---|---|
| JP | 02-307528 | A | 20 December 1990 | (Family: none) | |
| JP | 2006-022218 | A | 26 January 2006 | (Family: none) | |
| JP | 2004-337310 | A | 02 December 2004 | (Family: none) | |
| JP | 2006-061885 | A | 09 March 2006 | (Family: none) | |

Form PCT/ISA/210 (patent family annex) (July 2022)

**EP 4 732 816 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001323155 A

- JP H1133397 A